(19) Europäisches Patentamt
European Patent Office
Office européen des brevets

(11) Publication number: 0 386 683 A2

(12) EUROPEAN PATENT APPLICATION

(21) Application number: 90104229.1

(22) Date of filing: 05.03.90

(51) Int. Cl.5: C07D 473/04, C07D 473/06, A61K 31/52

(30) Priority: 10.03.89 IT 1973089

(43) Date of publication of application:
12.09.90 Bulletin 90/37

(84) Designated Contracting States:
AT BE CH DE DK ES FR GB GR IT LI LU NL SE

(71) Applicant: POLI INDUSTRIA CHIMICA S.p.A.
Piazza Agrippa, 1
I-20141 Milano(IT)

(72) Inventor: Poli, Stefano
Piazza Agrippa, 1
I-20141 Milano(IT)
Inventor: Del Corona, Lucio
Piazza Agrippa, 1
I-20141 Milano(IT)
Inventor: Coppi, Germano
Piazza Agrippa, 1
I-20141 Milano(IT)

(74) Representative: Minoja, Fabrizio
Studio Consulenza Brevettuale Via Rossini, 8
I-20122 Milano(IT)

# (54) Xanthine derivatives having bronchodilating activity, a process for the preparation thereof and pharmaceutical compositions containing them.

(57) Xanthine derivatives of general formula I

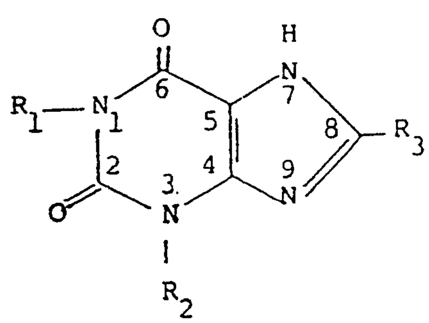

(I)

having valuable bronchodilating activity, a process for the preparation thereof and pharmaceutical compositions containing them.



Xerox Copy Centre

# XANTHINE DERIVATIVES HAVING BRONCHODILATING ACTIVITY, A PROCESS FOR THE PREPARATION THEREOF AND PHARMACEUTICAL COMPOSITIONS CONTAINING THEM.

The present invention relates to xanthine derivatives of general formula (I)

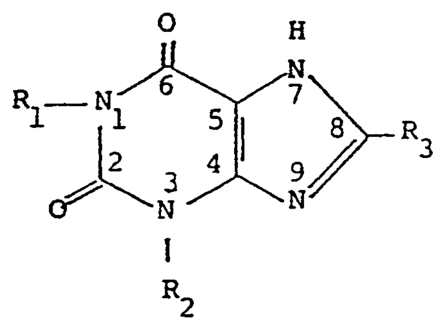

(I)

wherein $R_1$ is H or $C_1$-$C_6$ alkyl; $R_2$ is $C_1$-$C_6$ alkyl, $C_2$-$C_6$ alkoxyalkyl, $C_3$-$C_6$ cycloalkyl, $C_4$-$C_7$ cycloalkylmethyl; $R_3$ is H, $C_1$-$C_6$ alkyl, a $C_4$-$C_6$ aliphatic cyclic amino residue having one or more nitrogen atoms directly linked to the xanthine imidazole ring.

The present invention also relates to the process for the preparation of compounds of formula (I) and to pharmaceutical compositions containing them.

It has been found that changing $R_1$, $R_2$ and $R_3$ groups and above all introducing the cyclopropane cyclic group

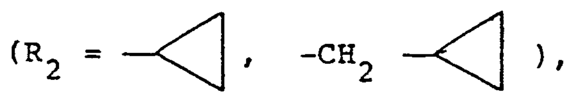

compounds having a remarkably higher bronchodilating activity than that of Theophylline can be obtained, which compounds, therefore, can advantageously be used in therapy to relieve or prevent bronchial asthma symptoms and for the treatment of bronchospasm associated with cronich bronchitis and emphysema.

The compounds of the present invention are prepared by conventional methods, e. g. according to scheme A:

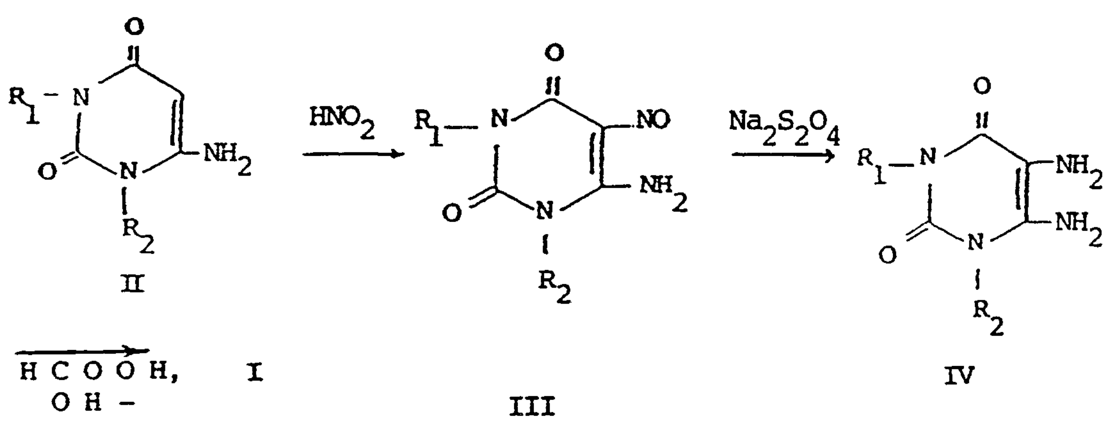


II III IV I

by nitrosating 6-aminouracil (II) (which is in its turn prepared by condensing the respective ureas with ethyl cyanoacetate in the presence of alkali alcoholates and alkylating with alkyl halides when $R_1$ # H), by means of an organic or sodium nitrite in alcoholic medium, in the presence of acids, reducing the resulting 5-nitroso-6-aminouracil (III) with sodium dithionite to give 5,6-diaminouracil (IV) which is then formylated with formic acid and treated with sodium hydroxide to give compounds (I).

The bronchodilating activity of the compounds of the present invention was evaluated by means of the

following tests, in which Theophylline was used as the control drug (I, $R_1 = R_2 = CH_3$ $R_3 = H$).

The compounds of the invention proved to be active at a dose of 10 mg/kg i.p. in the test of bronchoconstriction induced by intravenous administration of histamine in the guinea pig.

Male guinea pigs weighing 350-500 g, were anestethized with 60 mg/kg i.p. of 6% pentobarbital sodium, and artificially vented (pump by Basile: 50 cycles/min., 6.5 ml/cycle); the tracheal cannula was connected with a "bronchospasm transducer 7020" (Basile) modified method by Konzett and Roessler) and changes in respiratory volume were registered on a writing microdynamometer (Basile).

Bronchospasm was induced by 5 µg/kg i.v. of histamine, which caused a bronchoconstriction which is 80-90% the one obtained by complete occlusion of trachea. Such administration was repeated each 10 min. for a total of 3 times. 30 Seconds after each administration, air was insufflated into the lungs for 2 seconds.

The bronchodilating activities of the tested compounds are expressed as % change in bronchospasm obtained before treatment.

The data reported by way of example in Table 1 show that the activity of the novel compounds (ex. Ia and Ic) is substantially the same as the one of the standard drug.

Table 1.

| Antibronchospastic activity, after i.p. administration, in the test of bronchoconstriction induced in guinea pig by histamine i.v.(mean ± s.e. of 3 animals) | | |
|---|---|---|
| Compounds | Treatment | Maximum inhibition (%) |
| | (mg/kg/i.p.) | (time of max. effect) |
| Ia | 10 | 91,8 ± 2,2 (10′) |
| Ic | 10 | 97,6 ± 0,8 (10′) |
| Theophylline | 10 | 94,6 ± 1,1 (10′) |

also active, at a dose of 10 mg/kg i.p., in the tests of bronchoconstriction induced by acetylcholine (20 µg/kg/i.v.) and by bradykinin (20 µg/kg/i.v.).

The tests, which were similar to the above described one, showed that the novel compounds (ex. Ia) have a higher activity than that of the standard drug (see Table 2).

Table 2.

| Antibronchospastic activity, after i.p. administration, in the tests of bronchoconstriction induced by i.v. administration of acetylcholine and bradykinin in the guinea pig (mean ± s.e. of 3 animals) | | | |
|---|---|---|---|
| Compounds | Treatment | Maximum inhibition (%) | |
| | (mg/kg/i.p.) | (time of max. effect) | |
| | | Acetylcholine spasm | Bradykinin spasm |
| Ia | 10 | 92,3 ± 1,4 (5′) | 85,5 ± 5,6 (10′) |
| Theophylline | 10 | 48,8 ± 14,0 (5′) | 37,2 ± 1,3 (5′) |

Compounds of general formula (I) proved to be active also after oral administration in the test of bronchoconstriction induced by histamine in the guinea pig. Particularly, as shown in Table 3, compound Ia is markedly more effective than the standard drug as a bronchodilating agent.

Table 3.

| Antibronchospastic activity, after intraduodenal administration, in the test of bronchoconstriction induced by i.v. administration of histamine in the guinea pig (mean ± s.e. of 3 animals). | | |
|---|---|---|
| Compounds | Treatment (mg/kg/i.p.) | Maximum inhibition (%) (time of max. effect) |
| Ia | 31,6 | 86,0 ± 11,9 (30′) |
| | 100 | 91,7 ± 2,4 (30′) |
| Theophylline | 31,6 | 46,5 ± 14,2 (15′) |
| | 100 | 94,2 ± 1,7 (15′) |

The compounds of the present invention have acute toxicities equal or lower than that of Theophylline, by oral administration to the mouse.

The therapeutic index of the claimed compounds is more favourable than that of the control drug, due to the higher bronchodilating activity and the lower acute toxicity of the compounds of the invention.

Therefore, the compounds of the present invention can advantageously be used in therapy to relieve or prevent bronchial asthma symptoms and for the treatment of bronchospasm associated with chronic bronchitis and emphysema.

The present invention also relates to all of the industrially applicable aspects connected to the use of compounds of formula (I) as therapeutical agents. Therefore, an assential aspect of the invention is provided by pharmaceutical compositions containing, as the active ingredient, prefixed therapeutically effective amounts of one of compounds (I), besides possible excipients conventionally used in pharmaceutical technique. Examples of said pharmaceutical compositions comprise tablets, sugar-coated pills, hard- and soft-gelatin capsules, syrups, powders for the oral administration; lyophilized vials and solutions for the parenteral administration, and suppositories for rectal administration.

The single dosage for the oral, parenteral or rectal administration can range from 10 to 500 mg of one of compounds of formula (I), preferably from 25 to 200 mg.

The following examples further illustrate the present invention without limiting the spirit and scope thereof.

## EXAMPLE 1

3-Cyclopropylmethyl-xanthine

(I, $R_1 = R_3 = H$; $R_2$ =

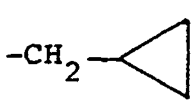

)

(compound Ia)

35g (0.307 mole) of cyclopropylmethylurea (prepared from 27 g of cyclopropylnitrile by reduction with sodium and alcohol) were added to a solution of 14.4 g (0.62 g. at.) of sodium in 288 ml of absolute ethanol and the mixture was heated to ebollition and added with 40.4 g (0.357 mole) of ethyl cyanoacetate in 100

ml of ethanol, during 2 hours. The reaction mixture was refluxed for 14 hours, then water was added thereto, ethanol was evaporated off under vacuum and the aqueous solution was acidified with acetic acid, cooled to 0° C and filtered, washing with chilled water. 35 g (62.9%) of 1-cyclopropylmethyl-6-aminouracil were obtained and dissolved in a mixture of 260 ml of ethanol, 525 ml of water and 1 ml of conc. hydrochloric acid. The solution was heated to 50° C and treated with 25 g (0.21 mole) of isoamyl nitrite. The reaction mixture was stirred for 30 min., cooled to 10° - 5° C, filtered and washed with water and ethanol to obtain 33 g (81.3%) of 1-cyclopropylme thyl-5-nitroso-6-aminouracil, which was suspended in 90 ml of ammonium hydroxide and treated with 66 g of sodium hydrosulfite at 40-50° C until decolorization. After filtration and drying, 29 g (93.6%) of 1-cyclopropylmethyl-5,6-diaminouracil were obtained. This compound was suspended in 175 ml of 85% formic acid, stirring for 2 hours at room temperature. By addition of ether and cooling to 0° C, 1-cyclopropylmethyl-5-amino-6-formylaminouracil precipitated which was suspended in 96 ml of 2N NaOH; the solution was heated to 90° C for 2 hours, cooled and acidified with acetic acid, to give 25.2 g (82.9%) of 3-cyclopropylmethylxanthine, m.p. 277.9° C, after crystallization from methanol.

| Analysis for $C_9H_{10}N_4O_2$ | | | |
|---|---|---|---|
| Calc.: | C% = 52.41 | H% = 4.88 | N% = 27.17 |
| Found: | C% = 52.26 | H% = 4.84 | N% = 27.07 |

$^1$H-NMR (DMSO-$D_2O$/TMS)
0.3-0.6 (m, 4H, -$CH_2$ cycl.); 1.3 (m, 1H, CH); 3.7-3.9 (m, 2H, $CH_2$); 7.9 (s, 1H, imid.); 10.8 (s, 1H, NH im); 13.4 (b.s., 1H, NH pyrim.)

By similar procedures, the compounds listed in Table 4 were obtained.

**TABLE 4**

| Compound | $R_1$ | $R_2$ | $R_3$ | Mp |
|---|---|---|---|---|
| Ib | H | $-CH_2-CH_2-O-CH_3$ | H | 157-8°C |
| Ic | H | $n-C_3H_7$ | 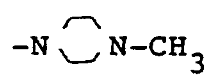 | (2.HCl) |
| Id | $n-C_6H_{13}$ | $n-C_6-H_{13}$ | N | 142-3°C |
| Ie | $n-C_5H_{11}$ | $n-C_3H_7$ | H | 198-200°C |
| If | H | 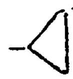 | H | 288-9°C |
| Ig | H | $-CH_2$ 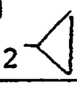 | H | 277-9°C |

## Claims

1. Compounds of general formula (I)

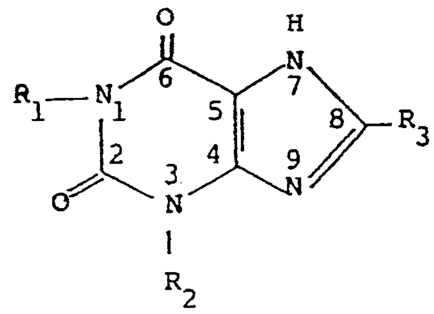

(I)

wherein $R_1$ is H or $C_1$-$C_6$ alkyl; $R_2$ is $C_1$-$C_6$ alkyl, $C_2$-$C_6$ alkoxyalkyl, $C_3$-$C_6$ cycloalkyl, $C_4$-$C_7$ cycloalkyl-methyl; $R_3$ is H, $C_1$-$C_6$ alkyl, a $C_4$-$C_6$ aliphatic cyclic amino residue having one or more nitrogen atoms.

2. A compound as claimed in claim 1, wherein $R_1$ = $R_3$ = hydrogen and $R_2$ = cyclopropylmethyl.

3. A compound as claimed in claim 1, wherein $R_1$ = $R_3$ = hydrogen and $R_2$ = methoxyethyl.

4. A compound as claimed in claim 1, wherein $R_1$ = hydrogen, $R_2$ = n=propyl and $R_3$ = 4-methyl-piperazin-1-yl.

5. A compound as claimed in claim 1, wherein $R_1$ = $R_2$ = n-hexyl and $R_3$ = hydrogen.

6. A compound as claimed in claim 1, wherein $R_1$ = n-pentyl, $R_2$ = n-propyl and $R_3$ = hydrogen.

7. A compound as claimed in claim 1, wherein $R_1$ = $R_3$ = hydrogen and $R_2$ = cyclopropyl.

8. A process for the preparation of the compounds as claimed in claims 1 to 7, which process comprises nitrosating 6-aminouracil (II) by means of an organic or sodium nitrite, reducing the obtained 5-nitroso-6-aminouracil (III) with sodium dithionite to give 5,6-diaminouracil (IV), which is finally formylated with formic acid and treated with sodium hydroxide to give compound (I).

9. Pharmaceutical compositions having bronchodilating activity, containing as the active ingredient one of the compounds of claims 1 to 7, in admixture with a pharmaceutically acceptable excipient.

10. Pharmaceutical compositions as claimed in claim 9, in form of tablets, gelatin capsules, powders, syrups, drops for the oral use; lyophilized vials and solutions for the parenteral use and suppositories for the rectal use.

11. The compounds as claimed in claims 1 to 7 for use as therapeutical agents.

12. The use of the compounds as claimed in claims 1 to 7 for the preparation of pharmaceutical compositions for the therapeutical treatment of bronchial asthma and bronchospasm associated with chronic bronchitis and emphysema.

Claim for the following Contracting States:ES,GR

1. A process for the preparation of compounds of general formula I

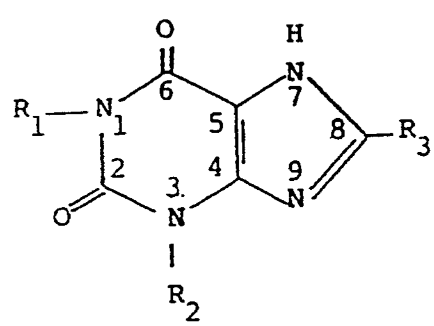

(I)

wherein $R_1$ is H or $C_1$-$C_6$ alkyl; $R_2$ is $C_1$-$C_6$ alkyl, $C_2$-$C_6$ alkoxyalkyl, $C_3$-$C_6$ cycloalkyl, $C_4$-$C_7$ cycloalkyl-methyl; $R_3$ is H, $C_1$-$C_6$ alkyl, a $C_4$-$C_6$ aliphatic cyclic amino residue having one or more nitrogen atoms, which process comprises nitrosating 6-aminouracil (II) by means of an organic or sodium nitrite, reducing the obtained 5-nitroso-6-aminouracil (III) with sodium dithionite to give 5,6-diaminouracil (IV), which is finally formylated with formic acid and treated with sodium hydroxide to give compound (I).